# EUROPEAN PATENT APPLICATION

(11) **EP 2 090 302 A1**
(43) Date of publication of application: **19.08.2009**
(21) Application number: 07828049.2
(22) Date of filing: 20.11.2007
(51) Int. Cl.: A61K 31/122, A23L 1/30, A61K 8/35, A61K 8/55, A61K 31/661, A61P 3/02, A61P 17/00, A61P 43/00, A61Q 19/00

(54) **DIETARY SUPPLEMENT, ANTI-FATIGUE AGENT OR PHYSICAL ENDURANCE ENHANCER, FUNCTIONAL FOOD, OR COSMETIC**

(30) Priority: 22.11.2006 JP 2006315874; 16.01.2007 JP 2007007013
(71) Applicant: Asahi Kasei Pharma Corporation, Tokyo 101-8481 (JP)
(72) Inventor: OTSUBO, Kazumasa, Tokyo 100-8440 (JP); KOGA, Shinji, Tokyo 100-8440 (JP); UENO, Yuji, Tokyo 100-8440 (JP); ISHIKAWA, Satoru, Tokyo 100-8440 (JP)
(74) Representative: Forstmeyer, Dietmar
(86) International application number: PCT/JP2007/001272
(87) International publication number: WO 2008/062559

(57) **Abstract**

Provided is an anti-fatigue agent or a physical endurance enhancer or a functional food having an anti-fatigue effect or a physical endurance-enhancing effect. Specifically provided are: a composition comprising phosphatidylinositol and coenzyme Q10; an anti-fatigue agent or a physical endurance enhancer comprising phosphatidylinositol and coenzyme Q10 as active ingredients; a functional food having an anti-fatigue effect or a physical endurance-enhancing effect, which comprises the compounds as active ingredients; use of phosphatidylinositol and coenzyme Q10 for the preparation of a composition having an anti-fatigue effect or a physical endurance-enhancing effect; and a method of enhancing an anti-fatigue effect or physical endurance, which includes ingesting a composition comprising those compounds as active ingredients.

## Description

### Technical Field

The present invention relates to a composition comprising phosphatidylinositol and coenzyme Q10; a dietary supplement comprising phosphatidylinositol and coenzyme Q10; an anti-fatigue agent and/or physical endurance enhancer comprising phosphatidylinositol and coenzyme Q10 as active ingredients; a functional food or cosmetic which contains phosphatidylinositol and coenzyme Q10 as active ingredients and has an anti-fatigue effect or physical endurance-enhancing effect; use of phosphatidylinositol and coenzyme Q10 for the purpose of preparing a composition having an anti-fatigue effect or a physical endurance-enhancing effect; and a method of enhancing an anti-fatigue effect or physical endurance including ingesting or applying a composition comprising phosphatidylinositol and coenzyme Q10 as active ingredients.

### Background Art

It has been reported that phosphatidylinositol is contained in soybean lecithin and is deeply involved in cell signaling. It has been reported in recent years that ingestion of phosphatidylinositol decreases a triacylglycerol (TG) concentration in blood and increases an HDL-C (high-density lipoprotein cholesterol) concentration (Non-patent Document 1), but other functions have not been reported.

On the other hand, coenzyme Q10 is a benzoquinone derivative and distributed widely in the living world, and thus it is named ubiquinone (oxidized coenzyme Q10). A hydroquinone compound obtained by reducing ubiquinone by two electrons is ubiquinol (reduced coenzyme Q10). There are many homologues with different lengths (n) of isoprenoid side chain (n=1 to 12). However, coenzyme Q10 is a biosynthesized product, and hence, major homologues are limited depending on the type of coenzyme Q10. In the case of mammals, the value of "n" is mainly 9 or 10. For example, in the case of a rat or mouse, the value is 9 in many cases, while in the case of a rabbit, the value is mainly 10. In the case of a human, the value is 10.

Coenzyme Q10 is a physiological component present as an electron transport system component of mitochondria in a cell of a living body and plays a role as a transport component in the electron transport system by repeating oxidation and reduction in the living body. Coenzyme Q10 has energy producing ability and antioxidant activity in the living body and is widely known to be useful. Coenzyme Q10 is a molecule obtained by biosynthesis in a human living body. It has been reported that the biosynthesis amount decreases with aging and the coenzyme Q10 level in the living body decreases due to various diseases. In the case of such diseases, external supply of coenzyme Q10 leads to good results. Moreover, it is considered that, not only in the case of a person suffering from a disease, but also in the case of an old person or a person with physical fatigue, it is necessary to supplement coenzyme Q10. Furthermore, in the living body, it is important to increase the ratio of reduced coenzyme Q10 having an antioxidant effect.

Oxidized coenzyme Q10 is used for medicinal application as a drug for congestive heart failure.
In addition to the medicinal application, it has also been reported that oxidized coenzyme Q10 is useful for geriatric diseases such as dementia or allergic diseases and enhances athletic performance. Moreover, extraneous supply of oxidized coenzyme Q 10 is considered to be an effective means because of its high safety, and many products containing reduced coenzyme Q 10 have been developed.

In recent years, owing to becoming more health conscious, functional foods having various efficacies have been developed using various natural materials. On the other hand, in order to keep good health positively and to increase the physical strength, sports have flourished more and more. In such background, studies have been made on basic concept of sports life and effects of dietary habits of health-conscious people and sports nutriology, and the studies draw increasing attention.

However, of various functional foods, there are no functional foods which are produced for the purpose of anti-fatigue and/or enhancement of physical endurance and have confirmed such effects. For example, a fatigue ameliorant containing coenzyme Q10 and carnitine (Patent Document 1) and a dietary supplement containing coenzyme Q 10, cyanocobalamine, folic acid, and pyridoxine hydrochloride (Patent Document 2) are known. [Patent Document 1] JP H07-330584 A
[Patent Document 2] JP 2003-169633 A
[Non-patent Document 1] Jim W. Burgess et al., Journal of Lipid Research (46) 350-355 (2005)

### Disclosure of the Invention

### Problems to be solved by the Invention

An object of the present invention is to provide a drug, functional food, or cosmetic which is superior to a conventional drug, functional food, or cosmetic having an anti-fatigue effect and/or physical endurance-enhancing effect.

### Means for solving the Problems

The inventors of the present invention have made extensive studies to solve the above-mentioned problems, and as a result, the inventors have found that simultaneous ingestion of phosphatidylinositol (the content of phosphatidylinositol in total phospholipids is equal to or above a certain level) and coenzyme Q10 can enhance an anti-fatigue effect or physical endurance-enhancing effect compared with single ingestion of phosphatidylinositol alone or coenzyme Q10 alone, thus completing the present invention related to a composition containing phosphatidylinositol and coenzyme Q10, a drug, functional food, or cosmetic each containing phosphatidylinositol and coenzyme Q10 as active ingredients.
Furthermore, in addition to the above, the inventors have found that phosphatidylinositol (the concentration of phosphatidylinositol in total phospholipids is a certain level or more) promotes absorption of coenzyme Q10 into the body, thereby completing the present invention.

That is, the present invention includes the following.

[A1] A composition including at least phosphatidylinositol and coenzyme Q10.

[A2] A composition according to the above [A1], in which the content of the phosphatidylinositol in total phospholipids is 50 mol% or more.

[A2-2] A composition according to the above [A1], in which the content of the phosphatidylinositol in total phospholipids is 85 mol% or more.

[A2-3] A composition according to the above [A1], essentially comprising phosphatidylinositol and coenzyme Q10, with the content of the phosphatidylinositol in total phospholipids being 50 mol% or more.

[A2-4] A composition according to the above [A1], essentially comprising phosphatidylinositol and coenzyme Q10, with the content of the phosphatidylinositol in total phospholipids being 85 mol% or more.

[A3] A composition according to any one of the above [A1] to [A2-4], in which the phosphatidylinositol is prepared by a method comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.
It should be noted that the case, in which the item numbers cited are indicated by the range such as [A1] to [A2-4] as described above and the item having a sub-number such as [A2-2] is included in the range, means that the item having the sub-number [A2-2] is also cited.

[A3-2] A composition according to any one of the above [A1] to [A2-4], in which the phosphatidylinositol is prepared by a method comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 85 mol% or more.

[A3-3] A composition according to any one of the above [A1] to [A2-4], in which the phosphatidylinositol is prepared by a method comprising at least the following step, and the composition contains no phosphatidylcholine, phosphatidylethanolamine, and phosphatidic acid other than the compounds derived from the following step of;
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

[A3-4] A composition according to any one of the above [A1] to [A2-4], in which the phosphatidylinositol is prepared by a method comprising at least the following step, and the composition contains no phosphatidylcholine, phosphatidylethanolamine, and phosphatidic acid other than the compounds derived from the following step of;
reacting a mixture of phospholipid derived from soybean with an enzyme which is derived from the genus Candida and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 85 mol% or more.

[A4] A dietary supplement comprising the composition according to any one of the above [A1] to [A3-4].

[A4-2] A dietary supplement essentially comprising the composition according to any one of the above [A1] to [A3-4].

[A5] A functional food comprising the composition according to any one of the above [A1] to [A3-4], the functional food having an anti-fatigue effect and/or a physical endurance-enhancing effect.

[A5-2] A functional food essentially comprising the composition according to any one of the above [A1] to [A3-4] and having an anti-fatigue effect and/or a physical endurance-enhancing effect.

[A6] A cosmetic comprising the composition according to any one of the above [A1] to [A3-4].

[A6-2] A cosmetic essentially comprising the composition according to any one of the above [A1] to [A3-4].

[A7] An anti-fatigue agent and/or a physical endurance enhancer comprising the composition according to any one of the above [A1] to [A3-4].

[A7-2] An anti-fatigue agent and/or a physical endurance enhancer essentially comprising the composition according to any one of the above [A1] to [A3-4].

[A8] An agent for promoting absorption of coenzyme Q 10 into a body, comprising at least phosphatidylinositol as an active ingredient.

[A9] An agent for promoting absorption of coenzyme Q10 into a body according to the above [A8], in which the content of the phosphatidylinositol in total phospholipids is 50 mol% or more.

[A9-2] An agent for promoting absorption of coenzyme Q10 into a body according to the above [A8], in which the content of the phosphatidylinositol in total phospholipids is 85 mol% or more.

[A9-3] An agent for promoting absorption of coenzyme Q10 into a body according to the above [A8], essentially comprising the phosphatidylinositol, in which the content of the phosphatidylinositol in total phospholipids is 50 mol% or more.

[A9-4] An agent for promoting absorption of coenzyme Q10 into a body according to the above [A8], essentially comprising the phosphatidylinositol, in which the content of the phosphatidylinositol in total phospholipids is 85 mol% or more.

[A10] An agent for promoting absorption of coenzyme Q10 into the body according to the above [A8] or [A9], in which the phosphatidylinositol is prepared by a method comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

[A10-2] An agent for promoting absorption of coenzyme Q10 into a body according to the above [A8] or [A9], in which the phosphatidylinositol is prepared by a method comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 85 mol% or more.

[A10-3] An agent for promoting absorption of coenzyme Q10 into a body according to any one of the above [A8] to [A9], in which the phosphatidylinositol is prepared by a method comprising at least the following step, the agent for promoting absorption of coenzyme Q10 into a body containing no phosphatidylcholine, phosphatidylethanolamine, and phosphatidic acid other than the compounds derived from the following step of;
reacting a mixture of phospholipid derived from soybean with an enzyme which is derived from the genus Candida and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

[A10-4] An agent for promoting absorption of coenzyme Q10 into a body according to any one of the above [A8] to [A9], in which the phosphatidylinositol is prepared by a method comprising at least the following step, the agent for promoting absorption of coenzyme Q10 into a body containing no phosphatidylcholine, phosphatidylethanolamine, and phosphatidic acid other than the compounds derived from the following step of;
reacting a mixture of phospholipid derived from soybean with an enzyme which is derived from the genus Candida and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 85 mol% or more.

[A11] A functional food having an effect of promoting absorption of coenzyme Q10 into a body, comprising at least phosphatidylinositol as an active ingredient.

[A12] A functional food having an effect of promoting absorption of coenzyme Q10 into a body according to the above [A11], in which the content of the phosphatidylinositol in total phospholipids is 50 mol% or more.

[A12-2] A functional food having an effect of promoting absorption of coenzyme Q10 into a body according to the above [A11], in which the content of the phosphatidylinositol in total phospholipids is 85 mol% or more.

[A13] A functional food having an effect of promoting absorption of coenzyme Q10 into a body according to the above [A11] or [A12], in which the phosphatidylinositol is prepared by a method comprising at least the following step of:
reacting a mixture of phospholipid derived from soybean with an enzyme which is derived from the genus Candida and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

[A13-2] A functional food having an effect of promoting absorption of coenzyme Q10 into a body according to the above [A11] or [A12], in which the phosphatidylinositol is prepared by a method comprising at least the following step of:
reacting a mixture of phospholipid derived from soybean with an enzyme which is derived from the genus Candida and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 85 mol% or more.

[A14] A kit comprising an anti-fatigue agent and/or a physical endurance enhancer containing phosphatidylinositol as an active ingredient and an anti-fatigue agent and/or a physical endurance enhancer containing coenzyme Q10 as an active ingredient in combination for the purpose of simultaneous ingestion of the phosphatidylinositol and the coenzyme Q10.

[A15] A kit comprising an anti-fatigue agent and/or a physical endurance enhancer containing phosphatidylinositol as an active ingredient and an anti-fatigue agent and/or a physical endurance enhancer containing coenzyme Q10 as an active ingredient in one package for the purpose of simultaneous ingestion of the phosphatidylinositol and the coenzyme Q10.

[A16] A kit according to the above [A14] or [A15], in which the content of the phosphatidylinositol in total phospholipids is 50 mol% or more.

[A16-2] A kit according to the above [A14] or [A15], in which the content of the phosphatidylinositol in total phospholipids is 85 mol% or more.

[A17] A method of preparing phosphatidylinositol comprising at least the following step of:
reacting a mixture of phospholipid derived from soybean with an enzyme which is derived from the genus Candida and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

[A17-2] A method of preparing phosphatidylinositol comprising at least the following step of:
reacting a mixture of phospholipid derived from soybean with an enzyme which is derived from the genus Candida and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 85 mol% or more.

[A17-3] A method of preparing phosphatidylinositol comprising the following steps 1) to 3):
1) reacting a mixture of phospholipid derived from soybean with an enzyme which is derived from the genus *Candida* and is substantially incapable of decomposing the phosphatidylinositol and is capable of selectively decomposing all of phospholipids such as phosphatidylcholine, phosphatidylethanolamine, phosphatidic acid, and phosphatidylserine to increase the content of the phosphatidylinositol in total phospholipids to 50 mol% or more;
2) efficiently extracting and collecting phosphatidylinositol using an organic solvent;
3) collecting the precipitates in a solvent in which phosphatidylinositol is not dissolved in an organic layer and impurities other than free fatty acids and phospholipids are dissolved.

[A17-4] A method of preparing phosphatidylinositol comprising the following steps 1) to 3):
1) reacting a mixture of phospholipid derived from soybean with an enzyme which is derived from the genus *Candida* and is substantially incapable of decomposing the phosphatidylinositol and is capable of selectively decomposing all of phospholipids such as phosphatidylcholine, phosphatidylethanolamine, phosphatidic acid, and phosphatidylserine to increase the content of the phosphatidylinositol in total phospholipids to 50 mol% or more;
2) efficiently extracting and collecting phosphatidylinositol using an organic solvent;
3) collecting the precipitates in a solvent in which phosphatidylinositol is not dissolved in an organic layer and impurities other than free fatty acids and phospholipids are dissolved; and
4) adding an antioxidant to an enzymatic reaction solution or during each production step of the phosphatidylinositol.

[A17-5] A method of preparing phosphatidylinositol according to the above [A17-3] or

[A17-4], in which in the step 2) the organic solvent is a mixture solvent of hexane and a lower alcohol.

[A17-6] A method of preparing phosphatidylinositol according to the above [A17-3] or [A17-4], in which in the step 2) the organic solvent is a mixture solvent of hexane and ethanol.

[A17-7] A method of preparing phosphatidylinositol according to any one of the above [A17-3] to [A17-6], in which in the step 3) the solvent is a lower alcohol, in which the phosphatidylinositol is not dissolved in the organic layer and impurities other than free fatty acids and phospholipids are dissolved.

[A 17-8] A method of preparing phosphatidylinositol according to any one of the above [A17-3] to [A17-6], in which in the step 3) the solvent is ethanol, in which the phosphatidylinositol is not dissolved in the organic layer and impurities other than free fatty acids and phospholipids are dissolved.

[A18] A use of phosphatidylinositol and coenzyme Q10 for preparing a food, a cosmetic, or an anti-fatigue agent and/or a physical endurance enhancer each having an anti-fatigue effect and/or a physical endurance-enhancing effect.

[A18-2] A use of phosphatidylinositol and coenzyme Q10 according to the above [A18], in which the content of the phosphatidylinositol in total phospholipids is 50 mol% or more.

[A18-3] A use of phosphatidylinositol and coenzyme Q10 according to the above [A18], in which the content of the phosphatidylinositol in total phospholipids is 85 mol% or more. [A19] A method of ameliorating fatigue and/or enhancing physical endurance comprising simultaneously ingesting phosphatidylinositol and coenzyme Q10.

[A19-2] A method of ameliorating fatigue and/or enhancing physical endurance according to the above [A19], in which the content of the phosphatidylinositol in total phospholipids is 50 mol% or more.

[A19-3] A method of ameliorating fatigue and/or a method of enhancing physical endurance according to the above [A19], in which the content of the phosphatidylinositol in total phospholipids is 85 mol% or more.

[A19-4] A method of ameliorating fatigue and/or enhancing physical endurance comprising previously ingesting phosphatidylinositol prior to simultaneous ingestion of phosphatidylinositol and coenzyme Q 10.

[A20] An alcohol metabolism-promoting agent comprising the composition according to any one of the above [1] to [3].

[A20-2] An agent for improving hepatic function comprising the composition according to any one of the above [1] to [3].

[B1] A dietary supplement comprising at least phosphatidylinositol and coenzyme Q10.

[B2] A dietary supplement according to the above [B1], in which the purity of phosphatidylinositol in total phospholipids is 50 mol% or more.

[B3] An anti-fatigue agent and/or a physical endurance enhancer comprising phosphatidylinositol as an active ingredient.

[B4] An anti-fatigue agent and/or a physical endurance enhancer according to the above [B3], further coprising coenzyme Q10 as an active ingredient.

[B5] An anti-fatigue agent and/or a physical endurance enhancer according to the above [B3] or [B4], further comprising a water-soluble substance.

[B6] An anti-fatigue agent and/or a physical endurance enhancer according to any of the above [B3] to [B5], in which the purity of the phosphatidylinositol in total phospholipids is 50 mol% or more.

[B7] A kit comprising an anti-fatigue agent and/or a physical endurance enhancer containing phosphatidylinositol as an active ingredient and an anti-fatigue agent and/or a physical endurance enhancer containing coenzyme Q10 as an active ingredient in combination for the purpose of simultaneous ingestion of the phosphatidylinositol and the coenzyme Q10.

[B8] A kit comprising an anti-fatigue agent and/or a physical endurance enhancer containing phosphatidylinositol as an active ingredient and an anti-fatigue agent and/or a physical endurance enhancer containing coenzyme Q10 as an active ingredient in one package for the purpose of simultaneous ingestion of the phosphatidylinositol and the coenzyme Q10.

[B9] A kit according to the above [B7] or [B8], in which the purity of the phosphatidylinositol in total phospholipids is 50 mol% or more.

[B10] A functional food having an anti-fatigue effect and/or a physical endurance-enhancing effect, comprising at least phosphatidylinositol and coenzyme Q10 as active ingredients.

[B11] A functional food according to the above [B10], in which the purity of the phosphatidylinositol in total phospholipids is 50 mol% or more.

[B12] A use of phosphatidylinositol and coenzyme Q10 for preparing a food, a cosmetic, or an anti-fatigue agent and/or a physical endurance enhancer each having an anti-fatigue effect and/or a physical endurance-enhancing effect.

[B13] A method of enhancing anti-fatigue effect and/or physical endurancecomprising simultaneously ingesting phosphatidylinositol and coenzyme Q10.

[B14] A cosmetic comprising at least phosphatidylinositol and coenzyme Q10 as active ingredients.

[B15] A dietary supplement according to the above [B1] or [B2], in which the phosphatidylinositol is obtained by reacting a phospholipid derived from soybean with an enzyme which has phospholipase B activity and is derived from the genus *Candida.*

[B16] An agent for promoting absorption of coenzyme Q10 into a body comprising phosphatidylinositol as an active ingredient.

[B17] A functional food having an effect of promoting absorption of coenzyme Q10 into a body comprising phosphatidylinositol as an active ingredient.

[B18] A method of preparing highly-pure phosphatidylinositol comprising the following steps 1) to 3):
1) reacting a phospholipid mixture with an enzyme which is substantially incapable of decomposing only phosphatidylinositol and has an effect of phospholipase B ;
2) efficiently extracting and collecting phosphatidylinositol using an organic solvent (such as hexane or ethanol); and
3) collecting the precipitates in a solvent (such as a lower alcohol) in which

phosphatidylinositol is not dissolved in an organic layer and impurities other than free fatty acids and phospholipids are dissolved.

[B19] A method of preparing highly-pure phosphatidylinositol comprising the following steps 1) to 3):
1) reacting a phospholipid mixture with an enzyme which is substantially incapable of decomposing only phosphatidylinositol and has an effect of phospholipase B;
2) efficiently extracting and collecting phosphatidylinositol using an organic solvent (such as hexane or ethanol); and
3) a step of separating and collecting phosphatidylinositol using an adsorbent (such as silica gel), to which the phosphatidylinositol is not adsorbed and impurities other than free fatty acids and phospholipids is adsorbed.

### Effect of the Invention

According to the present invention, it is possible to provide a dietary supplement comprising phosphatidylinositol and coenzyme Q10; an anti-fatigue agent and/or a physical endurance enhancer comprising phosphatidylinositol and coenzyme Q10 as active ingredients; a functional food or a cosmetic which comprises phosphatidylinositol and coenzyme Q10 as active ingredients and has an anti-fatigue effect and/or a physical endurance-enhancing effect; a use of phosphatidylinositol and coenzyme Q10 for preparing a composition having an anti-fatigue effect and/or a physical endurance-enhancing effect; and a method of enhancing anti-fatigue effect and/or physical endurance including ingesting a composition comprising phosphatidylinositol and coenzyme Q10 as active ingredients.

### Brief Description of the Drawings

FIG 1 shows a chromatography pattern of PLB of the present invention by gel filtration chromatography in Reference Example 4.
FIG 2 shows the optimum pH of PLB of the present invention in Reference Example 5.
FIG 3 shows the results of the pH stability of PLB of the present invention in Reference Example 5.
FIG 4 shows the results of the thermostability of PLB of the present invention in Reference Example 5.
FIG. 5 shows the results of isoelectric focusing electrophoresis of PLB of the present invention in Reference Example 5.
FIG. 6 shows the results of TLC in Reference Example 8.
FIG. 7 shows the results of TLC in Reference Example 9.

### Best Mode for carrying out the Invention

Hereinafter, the present invention will be described in detail.
According to the present invention, there is provided a composition comprising phosphatidylinositol and coenzyme Q10. Examples of the composition include, but not limited to, a nutritional composition and a pharmaceutical composition, and the composition is preferably a nutritional composition. In another aspect, the composition is preferably a pharmaceutical composition. The nutritional composition can be used for a food such as a dietary supplement or a functional food as described below, for example. Meanwhile, the pharmaceutical composition can be used in a medical product, for example.
The composition comprising phosphatidylinositol and coenzyme Q 10 of the present invention is not particularly limited as long as the content of phosphatidylinositol in total phospholipids in the composition is high. The lower limit of the content of phosphatidylinositol in total phospholipids in the composition is preferably 50 mol% or more, more preferably 60 mol% or more, still more preferably 70 mol% or more, still more preferably 80 mol% or more, still more preferably 85 mol% or more, and most preferably 90 mol% or more.
The contents of phosphatidylinositol and coenzyme Q10 in the composition of the present invention is an amount to achieve the ingestion amount of coenzyme Q 10 and phosphatidylinositol per day of 1 to 2,000 mg and 1 to 50,000 mg, preferably 10 to 500 mg and /10 to 10,000 mg, and still more preferably 30 to 300 mg and /50 to 1,000 mg, respectively.
That is, the ratio of coenzyme Q10/phosphatidylinositol is preferably 3/100 to 60 and particularly preferably 1/5 to 1.
The origin and production method of phosphatidylinositol to be used in the present invention are not particularly limited as long as phosphatidylinositol can be taken as a drug or eaten as a food. Phosphatidylinositol is desirably prepared from a soybean phospholipid by the hot ethanol method (JP 2000-300186 A), or from a soybean phospholipid using an enzyme having activity of phospholipase B (hereinafter, may be abbreviated as PLB) and being substantially incapable of reacting with phosphatidylinositol. The term "substantially incapable of reacting with phosphatidylinositol" or "substantially incapable of decomposing phosphatidylinositol" means that the upper limit of the ratio of the activity against phosphatidylcholine and the activity against phosphatidylinositol (the activity against phosphatidylinositol/the activity against phosphatidylcholine) is, for example, 15% or less, preferably 10% or less, more preferably 7% or less, still more preferably 5% or less, particularly preferably 3% or less, and most preferably 1% or less, while the lower limit is, for example, 0.0 1 % or more and preferably 0.1% or more.
Specifically, examples of the enzyme having the PLB activity and being substantially incapable of reacting with phosphatidylinositol include the following enzymes (1) to (4).
(1) An enzyme which is derived from the genus *Candida* and has the PLB activity and the following physicochemical properties:
   1) Reaction: To hydrolyze a phospholipid into 2-molar ratio of a free fatty acid
   2) Molecular weight: 53,000±3,000 (determined by SDS electrophoresis)
   3) Isoelectric point: pH 4.21±0.2
   4) Optimum pH: about pH 5.5 to 6.5
   5) pH stability: about pH 5 to 9 (treated at 37°C for 90 minutes)
   6) Stability: 55°C (treated at pH 5 for 10 minutes)
   7) Substrate specificity: the activity ratio against phosphatidylinositol is 10% or less compared with that against phosphatidylcholine.
(2) Phospholipase B containing enzyme fraction A or enzyme fraction B obtained by the following steps of:
   1) culturing *Candida cylindracea* (*rugosa*)
   2) concentrating the culture of *Candida cylindracea* (*rugosa*)
   3) precipitating an enzyme in an organic solvent
   4) purifying the crude enzyme solution obtained in the step 3) by hydrophobic chromatography
   5) separating and purifying the enzyme obtained in the step 4) into the enzyme fraction A or B by ion-exchange chromatography.
(3) Phospholipase B having the amino acid sequence of SEQ ID NO: 1
(4) Phospholipase B having the amino acid sequence of SEQ ID NO: 2

In addition to the above-mentioned enzymes, examples of the enzyme having the PLB activity and substantially incapable of reacting with phosphatidylinositol include an enzyme substantially incapable of decomposing the phosphatidylinositol and capable of decomposing all of phosphatidylcholine, phosphatidylethanolamine, and phosphatidic acid. The enzyme is preferably derived from the genus *Candida.*
Phospholipids are components of cell membranes and play important roles in physiological functions. In particular, phosphatidylinositol is deeply involved in signaling into or out of cells in cell membranes. The supply sources of the phospholipids to be used in drugs/foods are mainly soybean, hen egg yolk, and fish egg. These phospholipids are obtained as a mixture of plural molecular species including phosphatidylcholine (PC), phosphatidylethanolamine (PE), phosphatidylserine (PS), phosphatidic acid (PA), phosphatidylinositol (PI), and the like. As a method of separating highly-pure phosphatidylinositol from the phospholipid mixture, fractionation using an organic solvent, chromatography separation using a carrier such as silica gel or alumina or the like is known. PC and PE, which are present in a relatively high content, can be obtained efficiently by the above-mentioned methods, while a phospholipid component such as phosphatidylinositol, which is present in a low content, is inefficiently obtained by these methods. In addition, the methods require use of a large amount of a halogen-based organic solvent such as chloroform, thereby restricting the applications of the products. In order to efficiently produce phosphatidylinositol from phospholipids including PC, PE, PS, PA, PI, and the like, the phospholipids other than phosphatidylinositol (such as PC, PE, PA, and PS) may be selectively hydrolyzed so that phosphatidylinositol remains.

Specifically, phosphatidylinositol to be used in the present invention can be prepared by the following steps. That is, the phosphatidylinositol to be used in the present invention can be prepared by reacting a mixture of phospholipid derived from soybean with an enzyme having the PLB activity and being substantially incapable of reacting with phosphatidylinositol, for example, an enzyme which is substantially incapable of decomposing phosphatidylinositol and capable of decomposing all of phosphatidylcholine, phosphatidylethanolamine, and phosphatidic acid and is derived from the genus *Candida* to increase the content of phosphatidylinositol in total phospholipids to 50 mol% or more.
Meanwhile, for example, the thus-obtained phosphatidylinositol and coenzyme Q10 may be used to prepare the composition of the present invention. More specifically, the composition may be prepared by the following steps of: reacting soybean lecithin with the above-mentioned enzyme having the PLB activity and being substantially incapable of reacting with phosphatidylinositol; extracting phosphatidylinositol using an organic solvent (for example, a mixture solvent of hexane and ethanol) (for example, the pH during extraction is preferably in the range of pH 4.5 to 8.5); washing the extracted solvent with a buffer having pH 4 to 6, or mixing the extracting solvent with hydrous ethanol and then adjusting the pH to 4.0 to 5.0; collecting the precipitates in a solvent (for example, acetone or ethanol) in which phosphatidylinositol is not dissolved and impurities other than free fatty acids and phospholipids are dissolved; and drying and removing the solvent.

In addition, for the purpose of preventing oxidation of phosphatidylinositol during the production thereof, an antioxidant such as vitamin E (tocopherol) may be added in an enzyme reaction solution or in each production step. Examples of the antioxidant include L-ascorbic acid, sodium L-ascorbate, L-ascorbate, BHA, BHT, propyl gallate, erythorbic acid, sodium erythorbate, sodium sulfite (crystalline or anhydrous), and vitamin E (tocopherol).
The vitamin E (tocopherol) is preferred. The vitamin E is not particularly limited as long as the vitamin E is tocopherol. As the vitamin E, α-tocopherol, β-tocophenrol, γ-tocopherol, δ-tocopherol, and a mixture thereof are exemplified. The α-tocopherol is preferred. In addition, the γ-tocopherol may be preferred in some case. The addition amount of the vitamin E may be one capable of preventing the oxidation of phosphatidylethanol, and is preferably 0.0001% to 10% and more preferably 0.001% to 1%.

Phosphatidylinositol can be efficiently obtained by reacting the enzyme having the PLB activity and being substantially incapable of reacting with phosphatidylinositol, and selectively remaining only phosphatidylinositol in the phospholipid mixture. The phospholipid mixture may be previously dispersed in water using a homogenizer or the like, and a homogeneous aqueous solution may be prepared by the forcible stirring. The concentration of the phospholipids is not limited as long as the above-mentioned enzyme can react with the phospholipid mixture dispersed in water, and the concentration is in the range of 1 to 20%, preferably 5 to 10%, and particularly preferably 6 to 8%.

The pH in the reaction is not limited as long as the above-mentioned enzyme can react with the phospholipid mixture, and the pH is preferably adjusted to 3 to 10 and about 5.5 to 6.5 at which the activity of the enzyme reaches the highest. The pH is particularly preferably about 6. To keep the pH constant during the reaction, a buffer is preferably used. The type of the buffer is not particularly limited as long as the buffer has a buffering ability in the range of pH 5.5 to 6.5, and from the viewpoint of application to foods, an acetate buffer, citrate buffer, or phosphate buffer is particularly preferably used.

Further, the pH of the reaction solution may also be controlled to a preferable range by appropriately adding an alkaline solution during the reaction. To activate the enzymatic reaction, or to stabilize the enzyme, a calcium salt such as calcium chloride, calcium citrate, or calcium oxalate or a corresponding magnesium salt may be added.

The stirring rate during the enzymatic reaction has no problem as long as the enzymatic reaction proceeds well. For the purpose of emulsifying a substrate having poor solubility in water, a surfactant may also be added.

The enzyme having the PLB activity and being substantially incapable of reacting with phosphatidylinositol to be used in the present invention may be used in the concentration range of 1,000 to 100,000,000 units per kg of phospholipids, and preferably 2,000 to 5,000,000 units. The PLB used may be added in the form of a water-soluble product or may be used in the form immobilized on an insoluble carrier such as Celite or ion-exchange resin.

The enzymatic reaction temperature may be applied in a range where the above-mentioned enzyme is not deactivated. The upper limit of the temperature is preferably 60°C or less and more preferably 45°C or less, while the lower limit is preferably 10°C or more and more preferably 30°C or more.

The reaction time varies depending on the above-mentioned enzymatic reaction conditions and is usually 1 to 150 hours, and the reaction may be stopped based on the remaining amount of the substrate which is qualitatively determined. After completion of the reaction, the above-mentioned enzyme may be deactivated by a heat treatment or a pH treatment, which does not cause any problem. Silica-gel thin-layer chromatography (TLC) is the easiest method for confirming the state of the hydrolysis reaction . In the case where the remaining amount of the substrate is qualitatively determined using TLC by iodine coloring, the reaction is preferably stopped at the time when almost all spots showing phospholipids other than phosphatidylinositol are not observed on TLC. In the reaction product, free fatty acids, glycerylphosphorylcholine (GPC), glycerylphosphorylethanolamine (GPE), glycerophosphate (GP), remaining phosphatidylinositol which have not reacted, and the like exist by mixture.

To easily collect phosphatidylinositol from the mixture, there may be preferably employed a method of obtaining phosphatidylinositol from an organic layer obtained by adding a solvent in which phosphatidylinositol is dissolved and which contains an organic solvent that can be separated from water and then separating the solution. In the step of the solution separation operation, the mixture which has been brought into contact with the enzyme may be used as is, or the step of the liquid separation operation may be performed after a process of concentration or drying of the mixture.

Examples of the solvent used for extracting phosphatidylinositol include an organic solvent such as chloroform, ether, or hexane, and from the viewpoint of application to foods, the solvent containing an organic solvent that can be separated from water is preferably a mixture of n-hexane and a hydrophilic solvent. The hydrophilic solvent is particularly preferably a lower alcohol such as ethanol. Examples of the lower alcohol include methanol, ethanol, 1-propanol, and 2-propanol, and ethanol is preferable.

The lower limit of the volume of the water-soluble organic solvent is usually 10 parts by volume or more with respect to 100 parts by volume of the organic solvent that can be separated from water, preferably 20 parts by volume or more, and more preferably 30 parts by volume or more, while the upper limit is usually 400 parts by volume or less, preferably 300 parts by volume or less, more preferably 200 parts by volume or less, and particularly preferably 100 parts by volume or less, but not particularly limited thereto.

In the liquid separation operation after the extraction step, it is possible to separate the solution into the aqueous layer and the organic layer by allowing to stand as is, in addition to a mechanical separation operation such as centrifugation or the like. In the case of allowing to stand, for the purpose of obtaining sufficient separation of the aqueous layer and the organic layer containing phosphatidylinositol, the pH of the reaction solution may be changed before extraction. In this case, the pH is preferably adjusted to about 6.0 to 11, more preferably about 6.5 to 8.0.

Free fatty acids and phosphatidylinositol are soluble in an organic solvent, while GPC, GPE, GP and the like are insoluble in an organic solvent such as hexane but soluble in water.
As the temperature in the extraction operation and the subsequent standing operation, any temperature may be applied as long as the organic layer and the aqueous layer can be sufficiently separated. If the operations are performed at 20°C or more, preferably 40°C or more, and more preferably 50°C, good separation of the organic layer and the aqueous layer may be obtained.

To remove the free fatty acids efficiently, it is preferable to mix a mixture of an appropriate buffer having pH 5.0 or less and ethanol with an organic solvent to adjust the pH of the mixture to 5.0 or less (when the pH is lowered insufficiently, the pH is adjusted to 5.0 or less using an appropriate acid such as acetic acid, phosphoric acid or the like) after the phosphatidylinositol extraction operation. Meanwhile, the solvent is distilled off by concentrating the organic solvent layer under reduced pressure, and then the pH may be adjusted to 5 or less in the same manner as above.

Phosphatidylinositol and the free fatty acids which have been collected in the organic solvent layer may be concentrated under reduced pressure, for example, and the solvent is distilled off. Phosphatidylinositol is insoluble in a water-soluble or water-miscible alkylcarbonyl solvent or a lower alcohol solvent, while the free fatty acids are soluble in those solvents. Therefore, phosphatidylinositol can be collected as precipitates using these solvents. Examples of the water-soluble and water-miscible alkylcarbonyl solvent include acetone and methyl ethyl ketone, and examples of the lower alcohol solvent include ethanol, methanol, and propanol. Of those, acetone or ethanol is preferable. Ethanol is most preferable in order to simultaneously perform to remove the free fatty acids and remove unreacted materials or impurities (impurities other than phosphatidylinositol derived from the enzymatic reaction, e.g., glycolipids).

The temperature during the removal operation of the raw materials or impurities by ethanol is not limited as long as the removal is performed well, and the temperature is preferably 40°C or more and more preferably 50°C. The precipitates may be collected by adding 50°C-ethanol to a concentrated solution or concentrate, stirring the mixture well; keeping the temperature as it is or lowering the temperature to room temperature. The collection of phosphatidylinositol may be performed by either solid-liquid separation operation such as centrifugation or filtration.

Impurities other than phosphatidylinositol (such as glycolipids) may be removed using silica gel, to which phosphatidylinositol is adsorbed and the impurities other than phosphatidylinositol (such as glycolipids) is not adsorbed, after extraction of phosphatidylinositol with an organic solvent from the mixture obtained after completion of the enzymatic reaction, separation of the organic layer from the aqueous layer, and concentration of the organic solution. Meanwhile, raw materials or impurities may be separately collected by washing the concentrated solution with acetone then with ethanol, and concentrating and drying the ethanol-washed solution.

The phospholipid mixture is not particularly limited. There can be used phospholipids derived from hen egg yolk, phospholipids derived from fish egg such as salmon caviar, phospholipids derived from soybean, and the like. From the aspect of stable supply of raw phospholipids, the phospholipids derived from hen egg yolk or phospholipids derived from soybean is preferable, and the phospholipids derived from soybean is particularly preferable. The enzyme having the PLB activity and being substantially incapable of reacting with phosphatidylinositol, used in the present invention, preferably has high lipase activity in addition to the PLB activity. The fact that the enzyme has the lipase activity does not cause any problem in the production of phosphatidylinositol or glycerylphosphorylcholine from the soybean phospholipids. The soybean phospholipids are also supplied as a mixture with a triglyceride. In the case where the mixture is used as a raw material, it is significant for the enzyme to have both the lipase activity and PLB activity because the enzyme can hydrolyze the triglyceride and phospholipids simultaneously. In addition, in the case where the enzyme has high PLB activity and low or no lipase activity, the triglyceride and phospholipids may be simultaneously hydrolyzed by adding lipase separately.

According to the above-mentioned method of the present invention, highly-pure phosphatidylinositol is provided. The content of phosphatidylinositol is not particularly limited as long as phosphatidylinositol is highly-pure. The lower limit of the content of phosphatidylinositol in total phospholipids is preferably 50 mol% or more, more preferably 60 mol% or more, still more preferably 70 mol% or more, still more preferably 80 mol% or more, still more preferably 85 mol% or more, and most preferably 90 mol% or more. Highly-pure phosphatidylinositol is useful as a functional phospholipid.
Phosphatidylinositol has inositol including many hydroxyl groups in the molecule thereof. Therefore, it may be thought as advantages that phosphatidylinositol can be dispersed or dissolved in water well compared with other phospholipids such as PC, PE and the like, or phosphatidylinositol is extremely different in solubility or the like from the other lipid components, and phosphatidylinositol can thus be advantageously applied to a wide range as a phospholipid. The method of measuring the concentration of phosphatidylinositol usually includes thin-layer chromatography (TLC) and high-performance liquid chromatography (HPLC), of these, HPLC is preferably exemplified. As the HPLC, Standard Methods for the Analysis of Fats, Oils, and Related Materials (established by Japan Oil Chemists' Society, Standard Methods for the Analysis of Fats, Oils, and Related Materials, 2003) described in Reference Example 7 below can be given.

Specific examples of the above-mentioned enzyme having the PLB activity and being substantially incapable of reacting with phosphatidylinositol include the following enzymes (1) to (4):
(1) An enzyme having the PLB activity which is derived from microorganisms belonging to the genus *Candida,* wherein the enzyme has the following physicochemical properties:
   1) Reaction: To hydrolyze a phospholipid into 2-molar ratio of a free fatty acid and equimolar ratio of glycerylphosphorylcholine
   2) Molecular weight: 53,000±3,000 (determined by SDS electrophoresis)
   3) Isoelectric point: pH 4.21±0.2
   4) Optimum pH: about pH 5.5 to 6.5
   5) pH stability: about pH 5 to 9 (treated at 37°C for 90 minutes)
   6) Stability: 55°C (treated at pH 5 for 10 minutes)
   7) Substrate specificity: the activity against phosphatidylinositol is 10% or less compared with that against phosphatidylcholine.
(2) Phospholipase B containing enzyme fraction A and enzyme fraction B obtained by the following steps:
   1) culturing *Candida cylindracea* (*rugosa*) strain
   2) A step of concentrating the culture solution of *Candida cylindracea* (*rugosa*) cells
   3) A step of precipitating an enzyme with an organic solvent
   4) A step of purifying the crude enzyme solution obtained in the step 3) by hydrophobic chromatography
   5) A step of separating and purifying the enzyme obtained in the step 4) into the enzyme fraction A or B by ion-exchange chromatography.
(3) Phospholipase B having the amino acid sequence of SEQ ID NO: 1
(4) Phospholipase B having the amino acid sequence of SEQ ID NO: 2
   Those purified enzymes may be used, or cells or culture solution of having such activities, or crude purified products thereof may be used. In addition, use of a commercially-available enzyme having the PLB activity is easy and preferable.

The following method can determine whether the functional food, or anti-fatigue agent and/or physical endurance enhancer, which contains the composition of the present invention, has an anti-fatigue effect or not and physical endurance-enhancing effect or not. That is, the effect of the present invention can be determined as follows: after preliminarily feeding, attaching a weight, which is equivalent to 8.5% of the body weight of each mouse, to each mouse in a group subjected to forced oral administration with an anti-fatigue agent containing the composition of the present invention (administered group) and to each mouse in a group administered with no agent (non-administration group) three weeks after administration; forcing the mice to swim; measuring a time until the head (nose) of each mouse completely sinks in water for 7 seconds due to distress; and comparing the results between the administered group and the non-administration group.

The content of phosphatidylinositol in a drug or food is not limited as long as the anti-fatigue effect and/or physical endurance-enhancing effect can be obtained. The content may be adjusted to a level to achieve the ingestion amount of phosphatidylinositol per day of 1 to 50,000 mg, preferably 10 to 10,000 mg, and more preferably 50 to 1,000 mg.
The lower limit of the content of phosphatidylinositol used in the present invention in total phospholipids is preferably 50 mol% or more, more preferably 60 mol% or more, still more preferably 70 mol% or more, still more preferably 80 mol% or more, still more preferably 85 mol% or more, and most preferably 90 mol% or more.

The origin and production method of coenzyme Q10 used in the present invention are not limited as long as the coenzyme Q10 can be taken as a drug or eaten as a food, and commercially-available coenzyme Q10 is preferable. Coenzyme Q10 used in the present invention includes oxidized form or reduced form, and the oxidized coenzyme Q10 is preferable. In another aspect, the reduced coenzyme Q10 is preferable. Moreover, a compound which is converted into coenzyme Q10 in a living body may be used.

The content of coenzyme Q10 in a drug or food is not limited as long as an anti-fatigue effect and/or physical endurance-enhancing effect can be obtained. The content may be adjusted to a level to achieve the ingestion amount of coenzyme Q10 per day of 1 to 2,000 mg, preferably 10 to 500 mg, and more preferably 30 to 300 mg.

Coenzyme Q10 is a lipophilic solid with a low melting point, and coenzyme Q10 is known to exhibit low absorbability in oral administration because of its poor solubility in water. Therefore, it is preferable to improve the properties stability such as dispersion stability and anti-crystallization property and absorbability into the body of coenzyme Q10 in pharmaceutical preparations and foods. In the present invention, coenzyme Q10 previously stabilized its properties may be used. For example, coenzyme Q10 may be used after stabilizing coenzyme Q10 by blending coenzyme Q10 with an animal protein such as hen egg protein, milk protein, or meat protein, a vegetable protein such as soybean protein or soybean peptide, or hydrolysates thereof, or by further blending with carbohydrates, and improving the bioavailability.

In the case where coenzyme Q10 is used as an emulsified composition, for example, before use, the properties stability and bioavailability are improved by: dissolving coenzyme Q10 in a sucrose fatty acid ester such as sucrose acetate isobutyrate or a medium-chain fatty acid ester as an oil phase component, and emulsifying the solution with a polyhydric alcohol and an emulsifier; or dispersing coenzyme Q10, in the presence or absence of an additive such as an organic acid, in an aqueous substance such as gum arabic, agar, water-soluble corn fiber, starch, gelatin, xanthan gum, casein, dextrin, carmellose sodium, or polyvinylpyrrolidone and emulsifying the dispersed mixture.

The kit for simultaneous ingestion and/or application of phosphatidylinositol and coenzyme Q10 of the present invention is not limited as long as the kit includes an anti-fatigue agent and/or physical endurance enhancer comprising phosphatidylinositol as an active ingredient and an anti-fatigue agent and/or physical endurance enhancer comprising coenzyme Q10 as an active ingredient in combination, and the kit is preferably a kit including an anti-fatigue agent and/or physical endurance enhancer comprising phosphatidylinositol as an active ingredient and an anti-fatigue agent and/or physical endurance enhancer comprising coenzyme Q10 as an active ingredient in one package.

The drug, food, or cosmetic comprising phosphatidylinositol and coenzyme Q10 as active ingredients of the present invention shows at least an additive anti-fatigue effect and/or physical endurance-enhancing effect by their ingestion and/or application compared with the anti-fatigue effect and/or physical endurance-enhancing effect obtained by ingestion and/or application of only phosphatidylinositol or only coenzyme Q10. That is, the drug, food, or cosmetic for ameliorating fatigue and/or physical endurance is characterized by comprising phosphatidylinositol and coenzyme Q10 and having the effect of ameliorating fatigue and/or physical endurance.

The contents of phosphatidylinositol and coenzyme Q10 in a drug and food are not limited as long as the anti-fatigue effect and/or physical endurance-enhancing effect can be obtained. The content may be adjusted to achieve the ingestion amount of coenzyme Q10 and phosphatidylinositol per day of 1 to 2,000mg and 1 to 50,000 mg, preferably 10 to 500 mg and 10 to 10,000 mg, and still more preferably 30 to 300 mg and 50 to 1,000 mg, respectively.
That is, the ratio of coenzyme Q10/phosphatidylinositol is preferably 3/100 to 60 and particularly preferably 1/5 to 1.

The method of enhancing the anti-fatigue effect and/or physical endurance characterized by comprising ingesting the composition is also within the scope of the present invention. The ingestion includes both oral ingestion and parenteral ingestion, and the parenteral ingestion includes injection, intravenous drip, nasal ingestion, application to skin or scalp, or the like.

Meanwhile, the ingestion and/or application in the present invention may be performed by ingestion of a food or preparation comprising both phosphatidylinositol and coenzyme Q10 or by simultaneous ingestion of a food or preparation comprising only phosphatidylinositol and a food or preparation comprising only coenzyme Q10. Similarly, the ingestion and/or application may be performed by application of a cosmetic or preparation comprising both phosphatidylinositol and coenzyme Q10 or by simultaneous application of a cosmetic or preparation comprising only phosphatidylinositol and a cosmetic or preparation comprising only coenzyme Q10. The application and ingestion may be performed in combination.

The use of phosphatidylinositol and coenzyme Q10 of the present invention is not particularly limited as long as phosphatidylinositol and coenzyme Q10 is used for preparation of a food having the anti-fatigue effect or physical endurance-enhancing effect, or anti-fatigue agent and/or physical endurance enhancer of the present invention. A composition effective for the anti-fatigue effect and/or physical endurance-enhancing effect includes a drug serving as an anti-fatigue agent and/or physical endurance enhancer, and a functional food or cosmetic having the anti-fatigue effect and physical endurance-enhancing effect.

Phosphatidylinositol contained in total phospholipids at a certain concentration or above in the present invention may be used as an absorption-promoting agent for efficiently absorbing coenzyme Q10. The content of phosphatidylinositol in the absorption-promoting agent is not limited at all as long as the absorption-promoting effect can be obtained.
The content may be adjusted to a level to achieve the ingestion amount of phosphatidylinositol per day of 1 to 50,000 mg, preferably 10 to 10,000 mg, and still more preferably 50 to 1,000 mg.

According to the present invention, there is provided an alcohol metabolism-promoting agent comprising at least phosphatidylinositol and coenzyme Q10. In a preferred aspect, phosphatidylinositol used in the alcohol metabolism-promoting agent is used in the composition of the present invention.
The contents of phosphatidylinositol and coenzyme Q10 in the alcohol metabolism-promoting agent are not limited as long as the alcohol metabolism-promoting effect can be obtained. The contents may be adjusted to achieve the ingestion amounts of coenzyme Q10 and phosphatidylinositol per day of 1 to 2,000 mg and 1 to 50,000 mg, preferably 10 to 500 mg and 10 to 10,000 mg, and still more preferably 30 to 300 mg and 50 to 1,000 mg, respectively.
That is, the ratio of coenzyme Q10/phosphatidylinositol is preferably 3/100 to 60 and particularly preferably 1/5 to 1.

The effect of the alcohol metabolism-promoting agent of the present invention can be determined by the following method. That is, ethanol is administered to an ICR mouse, and 20 minutes after administration, the mouse is put on an accelerated rotor rod. Then, a time when the mouse remains on the rotating rotor rod (a time from when the mouse is put on the rotor rod to when the mouse drops) may be measured to determine the degree of lowered statokinetics function due to alcohol. If the time from the start of rotating the rotor rod to the drop of the mouse is long, it can be determined that the lowering of the balanced movement function is small, that is, alcohol metabolism has been promoted. The alcohol metabolism-promoting effect may be thought the hepatic function-improving effect.

The food comprising phosphatidylinositol of the present invention and coenzyme Q10 is not limited as long as the food contains those substances. Examples of the food include supplements comprising phosphatidylinositol and coenzyme Q10 (powder, granular, soft capsule, hard capsule, tablet, chewable tablet, rapidly-disintegrating tablet, syrup, liquid formulation, and the like), beverage including phosphatidylinositol and coenzyme Q10 (tea, carbonate beverage, fermented lactic-drink, sports drink, and the like), confectioneries comprising phosphatidylinositol (gummi candy, jelly, chewing gum, chocolate, cookie, candy, and the like), oil comprising phosphatidylinositol and coenzyme Q10, oil and fat foods comprising phosphatidylinositol (mayonnaise, dressing, butter, cream, margarine, and the like), ketchup comprising phosphatidylinositol and coenzyme Q10, sauce comprising phosphatidylinositol, liquid foods comprising phosphatidylinositol, dairy products comprising phosphatidylinositol and coenzyme Q10 (milk, yogurt, cheese, and the like), breads comprising phosphatidylinositol, noodles comprising phosphatidylinositol and coenzyme Q10 (wheat noodle (Udon), buckwheat noodle (Soba), Chinese noodle (Ramen), pasta, sauteed noodle (Yakisoba), flat white wheat noodle (Kishimen), the thinnest wheat noodle (Somen), thinner wheat noodle (Hiyamugi), rice noodle, and the like), and soup comprising phosphatidylinositol and coenzyme Q 10 (Miso soup, corn soup, consomme soup, and the like), and rice seasoning powder comprising phosphatidylinositol and coenzyme Q10.

The food comprising phosphatidylinositol and coenzyme Q10 used in the present invention may be blended, as required, with various nutrients, various vitamins (vitamin A, vitamin B1, vitamin B2, vitamin B6, vitamin B12, vitamin C, vitamin D, vitamin E, vitamin K, and the like), various minerals (magnesium, zinc, iron, sodium, potassium, selenium, titanium oxide, and the like), dietary fiber, various saccharides (cellulose, dextrin, chitin, and the like), various polyvalent unsaturated fatty acids (arachidonic acid, docosahexaenoic acid, eicosapentaenoic acid, docosapentaenoic acid, and the like), various conjugate fatty acids (conjugate linoic acid, conjugate linoleic acid, conjugate arachidonic acid, conjugate DHA, conjugate EPA, conjugate DPA, and the like), various phospholipids (lecithin, phosphatidic acid, phosphatidylserine, phosphatidylethanolamine, phosphatidylglycerol, phosphatidylcholine, phosphatidyl DHA, and the like), various glycolipids (cerebroside and the like), various carotinoids (β-carotene, lycopene, astaxanthin, β-cryptoxanthin, capsanthin, lutein, zeaxanthin, and the like), various flavonoids (quercetin, luteolin, isoflavon, and the like), various amino acids (glycin, serine, alanine, glutamine, valine, leucine, isoleucine, lysine, arginine, aspartic acid, glutamic acid, tryptophan, phenylalanine, histidine, proline, methionine, cysteine, and the like), other various nutrients (reduced coenzyme Q10, carnitine, sesamin, α-lipoic acid, inositol, D-chiro-inositol, pinitol, taurine, glucosamine, chondoroitin sulfate, S-adenosyl methionine, curcumin, γ-orizanol, glutathione, γ-aminobutyric acid, synephrine, pyrroloquinoline quinone, catechin, capsicin, and the like), stabilizing agents such as various dispersants and various emulsifiers, various sweeteners (sorbitol, sucrose, and the like) various gustatory components (citric acid, malic acid, and the like), flavor, royal jelly, honey, bees wax, propolis, agaricus, ginseng (*Panax ginseng C. A. Meyer*), bioperine, and the like. In addition, herbs such as peppermint, bergamot, chamomile, lavender and the like may be blended. In addition, materials such as theanine, dehydroepiandrosterone, and melatonin may be included.

The dietary supplement of the present invention includes a food for supplementing a nutrient that is insufficiently taken from only daily diet, and specific examples thereof include minerals and vitamins. The supplement further includes foods for enhancing spontaneous cure that human has intrinsically, improving immunity, preventing diseases, and supporting recovery from diseases.

Examples of the functional food of the present invention include a food with health claims, which is a health food and satisfies the standards specified by the country in consideration of the safeness and effectiveness. The functional foods are allowed to indicate the health claims and nutrient function claims, and are divided into "foods for specified health use" and "foods with nutrient function claims" depending on the purposes and functions of the foods.

The food comprising phosphatidylinositol and coenzyme Q10 of the present invention includes a kit including a food comprising phosphatidylinositol and a food comprising coenzyme Q 10 in combination, and a kit comprising both a food comprising phosphatidylinositol and a food comprising coenzyme Q10 in one package.

The drug comprising phosphatidylinositol and coenzyme Q10 of the present invention is not limited as long as the drug contains them. Examples of the dosage form of the drug include powders, granules, pills, soft capsules, hard capsules, tablets, chewable tablets, rapidly-disintegrating tablets, syrups, liquids, suspensions, suppositories, ointments, creams, gels, adhesive skin patches, inhalations, and injections. Those preparations may be prepared in accordance with conventional methods. However, phosphatidylinositol and coenzyme Q10 are poorly soluble in water, and hence, phosphatidylinositol and coenzyme Q10 may be used by dissolving in a nonhydrophilic organic solvent such as a vegetable oil or animal oil or dispersing and emulsifying in an aqueous solution together with an emulsifier, dispersant, surfactant or the like using a homogenizer (high-pressure homogenizer). In addition, in order to enhance the absorbability of phosphatidylinositol and coenzyme Q10, the compounds may be finely pulverized to an average particle size of about 1 µm in the presence or absence of an excipient (such as gum arabic, dextrin, casein or the like).

Examples of the additives that can be used in the preparation include soybean oil, safflower oil, olive oil, germ oil, sunflower oil, animal oils such as beef tallow and sardine oil, polyhydric alcohols such as polyethylene glycol, propylene glycol, glycerin, and sorbitol, surfactants such as sorbitan fatty acid ester, sucrose fatty acid ester, glycerin fatty acid ester, and polyglycerin fatty acid ester, excipients such as purified water, lactose, starch, crystalline cellulose, D-mannitol, maltose, lecithin, gum arabic, dextrin, sorbitol liquid, and saccharide liquid, sweeteners, colorants, pH adjusters, and flavors. In addition, the liquid formulation may be dissolved or suspended in water or other appropriate medium at dosing. Moreover, the tablet and granular may be coated by a known method.

In the case of injection administration, the drug is preferably administered intravenously, intraperitoneally, intramuscularly, subcutaneously, transdermally, intraarticularly, intrasynovially, intrathecally, subperiosteally, sublingually, or intraorally. In particular, intravenous administration or intraperitoneal administration is preferable. The intravenous administration may be either intravenous drip administration or porous administration.

Examples of the cosmetic comprising phosphatidylinositol and coenzyme Q10 of the present invention include creams, emulsions, lotions, micro-emulsion essences, poultices, and bath powders, and a fragrant material or the like may be blended therein.

### Examples

The present invention will be described based on preparation examples, food production examples, cosmetic production example, and test examples, but the present invention is not limited to the examples.
[Reference Example 1] Method of preparing an enzyme used in production of phosphatidylinositol, having activity of phospholipase B (PLB) and being substantially incapable of reacting with phosphatidylinositol
To a 30-liter-volume jar fermentor, added were 20 L of a sterilized medium containing 3% Pharmamedia (registered trademark) , 0.3% salt, 0.2% dipotassium phosphate, 0.2% monopotassium phosphate, 0.1% magnesium sulfate, and 0.3% antifoam agent, and 100 ml of a culture fluid of *Candida cylindracea* ATCC 14830 strain, precultured in the same medium (28°C, 4 days), was inoculated aseptically. The inoculated medium was aerated with sterile air at 20 L/min, and culture was performed at 28°C with stirring at 300 rpm. 50 hours later, the PLB activity reached the maximum value (0.2 U/ml). The resultant culture fluid was centrifuged at 5,000 rpm for 10 minutes to thereby obtain 16 L of the supernatant. The supernatant was concentrated by ultrafiltration. 9 L of cold acetone was added to 3 L of the resultant concentrate to produce precipitates, which were collected by centrifugation at 5,000 rpm for 10 minutes and dissolved in 2 L of 10 mM Tris-hydrochloric acid buffer (hereinafter, abbreviated as Tris-HCl) (pH 7.5) containing 2 M sodium chloride. The insoluble matters were removed by centrifugation, and the solution was passed through a column previously filled with Octyl-Sepharose (bed volume: 300 ml) to adsorb PLB. Subsequently, the column was washed with 10 mM Tris-HCl (pH 7.5) containing 2 M sodium chloride and 10 mM Tris-HCl (pH 7.5). PLB adsorbed to the column was eluted with 10 mM Tris-HCl (pH 7.5) containing 2% Adekatol SO120 (purification by hydrophobic chromatography). Then, the eluate (1 L) was passed through a DEAE-Sepharose column (bed volume: 200 ml) previously equilibrated with 10 mM Tris-HCl (pH 7.5) to adsorb PLB, and the column was washed with the above-mentioned buffers, followed by elution of the PLB enzyme with a concentration gradient of 0 to 0.3 M sodium chloride. PLB enzyme fraction A and PLB enzyme fraction B were obtained at a sodium chloride- level of about 0.1 M and about 0.25 M, respectively (separation and purification by ion-exchange chromatography).
The N-terminal amino acid sequences of the enzyme fractions A and B were determined by an amino acid sequencer, and the amino acid sequences of the enzyme fractions A and B were found to be the sequences of SEQ ID NOS: 1 and 2, respectively. The homology of the enzyme fraction B to the enzyme fraction A was analyzed by a homology analysis using GENETEX WIN 5.2 (manufactured by Software Co., Ltd.), and the homology was found to be 88.5%.

### [Reference Example 2] Method of measuring PLB activity

The enzymatic activity of PLB was measured GPC produced from lecithin by the enzyme method. Specifically, 0.5 ml of a reaction solution obtained by dissolving 0.05 ml of 10 mM egg-yolk phosphatidylcholine, 0.025 ml of 1 M calcium chloride, 0.05 ml of 0.2% TODB, 0.05 ml of 0.2% 4-aminoantipyrine, 0.1 ml of 50 U/ml monoglyceride lipase, 0.1 ml of 300 U/ml glycerophosphate oxidase, 0.025 ml of 6 U/ml GPC phosphodiesterase, and 0.05 ml of 100 U/ml peroxidase in 0.05 ml of 1 M MES-NaOH buffer (hereinafter abbreviated as MES-NaOH) (pH 6) containing 3% Triton X100 was preliminarily heated at 37°C for 2 to 3 minutes, and 25 µl of PLB solutions (0.03 to 0.15 U/ml) prepared by dissolving in 10 mM Tris-HCl (pH 7.5) containing 0.05% BSA and diluting with the same buffer were added to the reaction solution to start the reaction. After exactly 10 minutes, 1 ml of 0.5% SDS was added to stop the reaction, and absorbance at 550 nm was measured.
Note that one unit of the PLB activity was defined as an activity to release 1 µMol GPC per minute.

### [Reference Example 3] Method of measuring lipase (LP) activity

The lipase activity was measured by the enzyme method after conversion of a monoglyceride produced using a diglyceride as a substrate into glycerin with monoglyceridelipase. Specifically, 25 µl of enzyme solutions (0.03 to 0.15 U/ml), prepared by diluting the enzyme with 10 mM Tris-HCl containing 0.05% BSA, were added to 0.5 ml of a reaction solution obtained by dissolving 0.05 ml of 10 mM 1,2-diglyceride, 0.025 ml of 0.05 M calcium chloride, 0.025 ml of 0.05 M magnesium chloride, 0.05 ml of 0.05 M ATP, 0.05 ml of 10 U/ml monoglyceridelipase, 0.05 ml of 5 U/ml glycerokinase, 0.025 ml of 400 U/ml glycerophosphate oxidase, 0.025 ml of 100 U/ml peroxidase, 0.025 ml of 0.3% TOOS, and 0.025 ml of 0.3% 4-aminoantipyrine in 0.1 ml of 1 M MES-NaOH (pH 6.0) containing 3% Triton X100, and the whole was reacted at 37°C for 10 minutes. Then, 0.5% SDS was added to stop the reaction, and absorbance at 550 nm was measured. Note that one unit of the lipase activity was defined as an activity to release 1 µMol glycerin per minute.

### [Reference Example 4] Purification of PLB enzyme fraction A by gel filtration chromatography

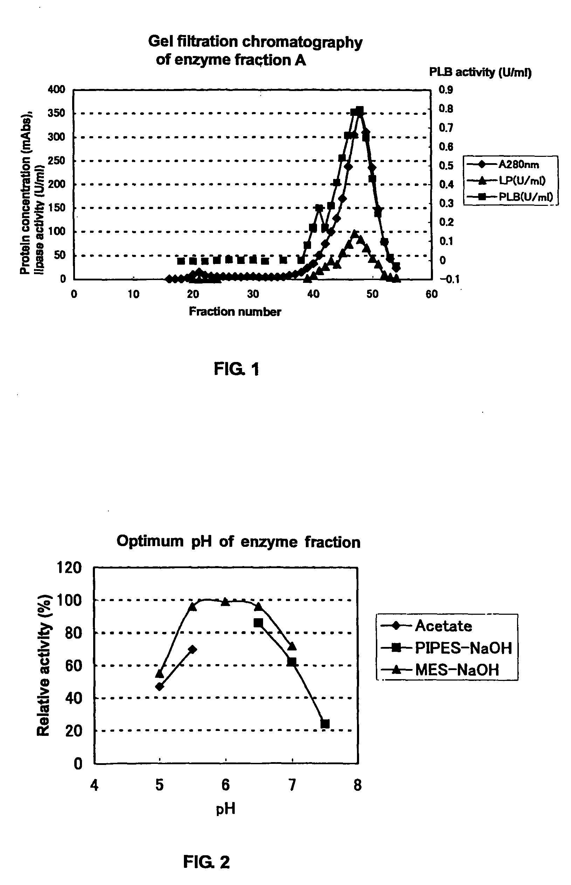

PLB enzyme fraction A obtained in Reference Example 1 was concentrated using a centrifugal-type ultrafiltration apparatus. The concentrated solution was separated by gel filtration chromatography (Superdex 75) preliminarily equilibrated with 10 mM Tris-HCl (pH 7.5) containing 0.5 M sodium chloride at a flow rate of 0.5 ml/min, to thereby obtain active fractions.
The PLB activity and LP activity in each fraction were determined by the methods of Reference Examples 2 and 3, and the absorbance at 280 nm was measured to determine the protein concentration. The results of the PLB activity, LP activity, and protein concentration in each fraction are shown in FIG 1.
The results reveal that the protein concentration measured at 280 nm (in FIG. 1, shown as A280 nm) and the LP and PLB activities show the same elution pattern, and the protein having the LP activity and the protein having the PLB activity were found to be the same enzyme protein.

### [Reference Example 5] Properties of PLB

### 5-1 (Optimum pH in the reaction)

The PLB enzymatic activity of PLB enzyme fraction A was measured at different pH values in the same way as in Reference Example 2 except that the buffer in the composition of the reaction solution was replaced by an acetate buffer (hereinafter, abbreviated as Acetate), a phosphatidylinositol PES-NaOH buffer (hereinafter, abbreviated as phosphatidylinositol PES-NaOH), or MES-NaOH, and the relative activity in each buffer to the activity when using MES-NaOH (pH 6.0) was calculated. The results are shown in FIG 2.
As shown in FIG. 2, in the case of MES-NaOH, the enzymatic activity reaches the maximum value at about pH 6, and in the cases of phosphatidylinositol PES-NaOH and Acetate, the enzymatic activity is anticipated to reach the maximum value at about pH 6 as well.

### 5-2 (pH stability)

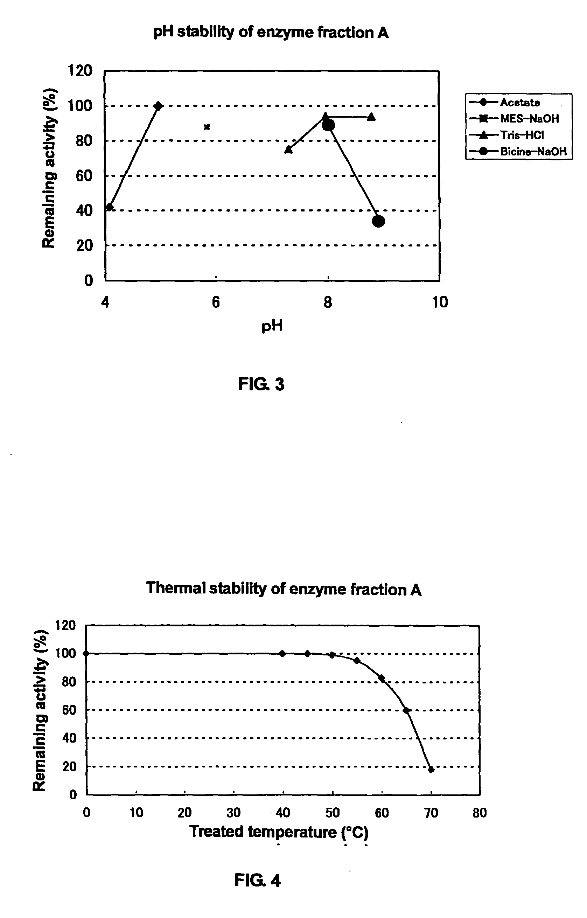

PLB enzyme fraction A was dissolved in each buffer of a 10 mM Acetate, 10 mM MES-NaOH, 10 mM Tris-HCl, or a 10 mM Bicine-NaOH buffer (hereinafter, abbreviated as Bicine-NaOH) so as to have 5 U/ml, and the solutions were allowed to stand at 37°C for 90 minutes. Then, the PLB activity was measured based on the method in Reference Example 2, and relative remaining activities at different pH values to the activity in Acetate (pH 5) were calculated. The results are shown in FIG 3.
As shown in FIG. 3, PLB was found to be stable in a wide range from pH 5 to pH 8.

### 5-3 (Thermostability)

Enzyme fraction A was dissolved in 10 mM MES-NaOH (pH 6.0) to have 5 U/ml, and the solution was heated by the same method in Reference Example 2 at a temperature ranging from 0 to 70°C for 10 minutes, followed by measurement of the PLB activity. The results are shown in FIG. 4. When the PLB activity of refrigerated enzyme fraction A is defined as 100%, the relative remaining activities were 90% or more for the treatments up to 55°C. Therefore, PLB was found to be a heat-stable enzyme.

### 5-4 (Substrate specificity)

The relative activities of two kinds of PLBs (enzyme fraction A and enzyme fraction B) obtained by ion-exchange chromatography in Reference Example 1 for different phospholipids with respect to phosphatidylcholine (PC) were measured by the PLB activity measurement method described in Reference Example 2. The results are shown in Table 1.
The results reveal that, in both cases of enzyme fraction A and enzyme fraction B, the relative activities for phosphatidylinositol (PI) with respect to PC are particularly lower than those for the other phospholipids. Therefore, PLB enzyme fraction A and PLB enzyme fraction B were found to have substrate specificity that the fractions had low activities for PI among phospholipids. As shown in Table 1, both the fractions have PLB activities, and hence, a mixture of the fractions may also be used as PLB.

**[Table 1]**

| Substrate | Relative activity (%) | |
|---|---|---|
| | Enzyme fraction A | Enzyme fraction B |
| Phosphatidylcholine (PC) | 100 | 100 |
| Phosphatidylethanolamine (PE) | 32.1 | 45.8 |
| Phosphatidic acid (PA) | 20.1 | 30.5 |
| Phosphatidylinositol (PI) | 5.4 | 4.2 |

### 5-5 (Molecular weight)

Enzyme fraction A obtained by the method described in Reference Example 1 was analyzed by SDS polyacrylamide gel electrophoresis, and a single band was observed. The molecular weight obtained using a protein having a known molecular weight as a marker was 53 kDa.

### 5-6 (Isoelectric point)

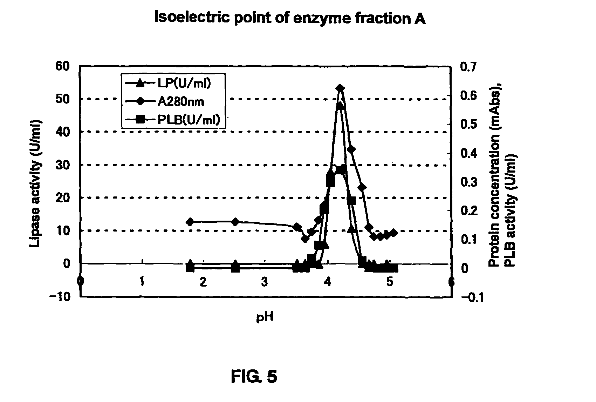

The isoelectric point of enzyme fraction A obtained by the method described in Reference Example 1 was measured by isoelectric point electrophoresis in a pH gradient generated using carrier ampholytes, and the protein concentration was measured by absorbance at 280 nm. The relationship between the isoelectric point and enzyme concentration in enzyme fraction A is shown in FIG. 5. As a result, the pH shown by the protein measured by the absorbance at 280 nm and the lipase activity and PLB activity were completely correspondent, and the pH value (isoelectric point) was found to be 4.21. As can be seen from FIG. 5, the peaks of PLB, lipase, and the protein (the peak shown at A280 nm in FIG. 5) were completely the same, and hence, it was clarified that PLB and LB were the same enzyme protein.

### [Reference Example 6] Method of quantifying phosphatidylinositol (PI)

0.02 ml of PI solution obtained by dissolving test samples containing phosphatidylinositol (PI) (1 to 3 mg/ml) in 2% Triton X100 was added to 0.5 ml of a coloring solution consisting of 0.1 ml of 1 M Tris-HCl (pH 8), 0.05 ml of 10 mM NAD, 0.05 ml of 10 mM magnesium chloride, 0.05 ml of 2% Triton X100, 0.05 ml of 50 U/ml phosphatidylinositol-specific phospholipase C, 0.05 ml of 50 U/ml alkaline phosphatase, 0.05 ml of 50 U/ml inositol dehydrogenase, 0.05 ml of 5% nitrotetrazolium blue, and 0.15 ml of purified water to perform a reaction at 37°C for 10 minutes. The reaction was stopped with 0.5 ml of 0.5% SDS, and absorbance at 550 nm was measured. PI was quantified using an aqueous solution of a known concentration of inositol as a calibrator.

### [Reference Example 7] Method of preparing phosphatidylinositol (PI)

1) 40 g of a phospholipid derived from soybean was suspended in 400 ml of 10 mM acetate buffer (pH 5.8) with stirring, and 1,400 units of PLB (enzyme fraction A obtained in Reference Example 1) was added thereto to start a reaction at 45°C. After completion of the reaction, NaOH was added to improve lowered flowability of the solution due to solid matters formed by the reaction, and the pH was adjusted to 7.5.
2) After 20 hours, the reaction was stopped, and 200 ml of ethanol and 400 ml of hexane were added, followed by stirring at 50°C for 1 hour. The hexane layer and aqueous layer were separated, and the organic solvent layer was collected.
3) To the organic layer added was a mixture of 300 ml of 10 mM acetate buffer (pH 4.5) and 300 ml of ethanol, and the pH was adjusted to 5.0 with acetic acid. The resultant solution was stirred at 50°C for 1 hour and allowed to stand for 1 hour, and the hexane layer and aqueous layer were separated.
4) The collected organic solvent was concentrated under reduced pressure, and 400 ml of ethanol was added to the concentrate, followed by stirring at 50°C for 1 hour. Then, the precipitates were collected by filtration, and 400 ml of ethanol was further added, followed by the same procedures as above. The resultant precipitates were collected and dried, thereby obtaining 6.2 g of powder containing a high concentration of phosphatidylinositol. The purity of the resultant phospholipid was measured by Standard Methods for the Analysis of Fats, Oils, and Related Materials (established by Japan Oil Chemists' Society, Standard Methods for the Analysis of Fats, Oils, and Related Materials, 2003), and was 88.9 mol% in total phospholipids.

### [Reference Example 8] Production method excluding step 3) in Reference Example 7

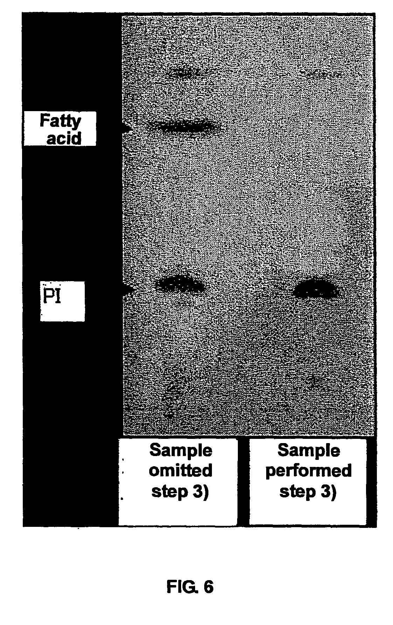

FIG. 6 shows the results of a TLC analysis for phosphatidylinositol obtained by concentrating the organic solvent layer, washing the solution with ethanol, and filtrating and drying the precipitates without performing washing of the organic solvent layer with a mixture solvent including an acetate buffer and ethanol in the step 3) of Reference Example 7. The results reveal that free fatty acids can be completely removed by the performance of the step 3).

### [Reference Example 9] Production method using acetone instead of ethanol in step 4) of Reference Example 7

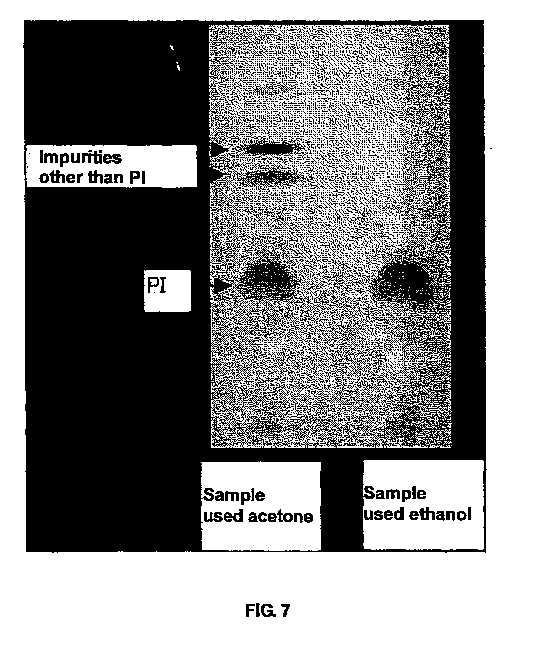

FIG. 7 shows the results of a TLC analysis for phosphatidylinositol obtained by using acetone instead of ethanol in the step 4) of Reference Example 7. The results reveal that, if ethanol is used, impurities other than phosphatidylinositol (glycolipids and the like) can be completely removed.

### [Reference Example 10] Production method including removing impurities by silica gel instead of step 4) in Reference Example 7

The organic solvent collected after the step 3) in Reference Example 7 was concentrated under reduced pressure, and 400 ml of hexane was added thereto. The solution was charged to a silica gel equilibrated with 400 ml of hexane, and a phosphatidylinositol fraction, which was passed through the gel, was collected, concentrated, and dried. The resultant sample was subjected to a TLC analysis, and it was found that highly-pure phosphatidylinositol containing no impurities other than phosphatidylinositol (glycolipids and the like) was obtained as in the case of Reference Example 7 where ethanol was used.

**[Preparation Example 1]**

| <Tablet> | |
|---|---|
| Phosphatidylinositol | 120 g |
| Coenzyme Q10 | 120 g |
| Crystalline cellulose | 330 g |
| Carmellose-calcium | 15 g |
| Hydroxypropyl cellulose | 10 g |
| Purified water | 60 ml |

The components were blended and dried by a conventional method so that the above-mentioned composition is achieved, and 10 g of magnesium stearate is added thereto, followed by tableting, thereby obtaining 100 mg of tablets containing 20 mg of phosphatidylinositol and 20 mg of coenzyme Q10.

### [Preparation Example 2]

### <Soft capsule>

200 g of phosphatidylinositol and 200 g of coenzyme Q10 are added in 200 g of olive oil and dissolved at about 60°C, and the solution is stirred uniformly and cooled, thereby obtaining a coenzyme Q10-containing material. Gelatin (70%) and glycerin (30%) are blended to swell the material, and to form a dissolved gelatin sheet. The gelatin sheet is filled with the coenzyme Q10-containing material as a content solution so that the amount of the material per capsule is 300 mg, followed by drying, and soft capsules containing 100 mg of phosphatidylinositol and 100 mg of coenzyme Q10 can be thus obtained.

### [Kit Example]

The above-mentioned procedures are repeated except that a tablet or soft capsule containing only phosphatidylinositol or only coenzyme Q10 is prepared instead of the tablet described in Preparation Example 1 or soft capsule described in Preparation Example 2, each of which contains both phosphatidylinositol and coenzyme Q10, to thereby obtain a preparation (tablet or soft capsule). A kit including both phosphatidylinositol and coenzyme Q10 in one package can be produced by packing one to five of the tablets (or soft capsules) containing only phosphatidylinositol and one to five of the tablets (or soft capsules) containing only coenzyme Q10 in one package.

### [Food Production Example 1]

### <Drink>

2,500 g of phosphatidylinositol and 500 g of coenzyme Q10 were dissolved in 10 g of glycerin fatty acid ester and 30 g of glycerin heated to 60°C. Water was added to the mixture up to 100 ml, and the solution was subjected to emulsification treatment using an emulsifier (pressurized homogenizer), to thereby obtain an emulsified composition. Water was added to 1 g of the emulsified composition up to 100 ml, followed by stirring, and then a drink containing phosphatidylinositol (250 mg/100 ml) and coenzyme Q10 (50 mg/100 ml) can be obtained.

### [Food Production Example 2]

### <Bread>

1 g of phosphatidylinositol, 1 g of coenzyme Q10, 15 g of sugar, 2 g of salt, and 5 g of dried skim milk are dissolved in 70 g of hot water, and two hen eggs are added, followed by mixing well. The mixture is added to a mixture obtained by mixing 130 g of flour and 2 g of dry yeast, and the whole is kneaded by hand. Then, about 30 g of butter is added thereto, and the whole is further kneaded, to thereby produce 30 bread roll doughs. Subsequently, fermentation is performed, and the surfaces of the doughs are coated with beaten egg. The resultant doughs are baked at 180°C for 15 minutes, to thereby obtain bread rolls.

### [Food Production Example 3]

### <Udon (Japanese wheat noodle)>

200 g of water, 2 g of phosphatidylinositol, 2 g of coenzyme Q10, and 20 g of salt are added to 400 g of flour, and the whole is kneaded well and allowed to stand. Then, the dough is stretched and cut at a width of about 6 mm, to thereby obtain udon.

### [Food Production Example 4]

### <Drink containing phosphatidylinositol and coenzyme Q10>

30 g of coenzyme Q10 and 30 g of phosphatidylinositol obtained in Reference Example 7 are suspended in a 5-fold volume of olive oil, and the suspension is heated to 50°C, to thereby obtain an oil phase. 10 g of glycerol fatty acid ester as an emulsifier is added to 90 g of glycerin, and the whole is heated to 70°C to dissolve. The above-mentioned oil phase is gradually added to the solution with stirring. The mixture is subjected to high-pressure emulsification treatment using an emulsifier, to thereby obtain an emulsified composition. To 20 g of the emulsified composition was added 180 ml of water, followed by stirring, to thereby obtain a drink containing phosphatidylinositol and coenzyme Q10.

### [Cosmetic Production Example 1]

### <Cream>

Appropriate amounts of coenzyme Q10, phosphatidylinositol, stearyl alcohol, propylene glycol, sorbitol, paraben, vitamin E, a flavor, and purified water are blended in accordance with a conventional method, and a cream may be thus obtained.
Note that, in the cases of Preparation Examples 1 and 2, Kit Example, Food Production Examples 1 to 4, and Cosmetic Production Example 1, the products can be prepared using, as coenzyme Q10, oxidized coenzyme Q10 or reduced coenzyme Q10.

### [Test Example 1] Test of anti-fatigue effect and physical endurance-enhancing effect

Six-week-old male Slc:ddY mice (Charles river laboratories Japan, Inc.) were preliminarily fed for 7 days and divided into four groups each including 10 mice.
The groups include: the group administered with water (control); the group administered with oxidized coenzyme Q10 at 50 mg/kg/day (Comparative Example 1, group administered with only CoQ10); the group administered with phosphatidylinositol at 100 mg/kg/day (Comparative Example 2, group administered with only phosphatidylinositol); and the group administered with oxidized coenzyme Q 10 at 50 mg/kg/day and phosphatidylinositol at 100 mg/kg/day at the same time (Test Example, group administered with CoQ10 + phosphatidylinositol), and forced oral administration was performed under the above-mentioned administration conditions five days a week. Three weeks after the administration, a weight was attached to each mouse (the weight is 8.5% of the body weight of the mouse), and the mouse was forced to swim. Then, a time until the head (nose) of the mouse completely sank for 7 seconds due to distress was measured. The results are shown in Table 1. Note that, the oxidized coenzyme Q10 and phosphatidylinositol in Test Example were simultaneously administered. As shown in Table 2,the group administered with phosphatidylinositol and coenzyme Q10-containing food (Test Example) was admitted to exhibit significantly longer effect in the swimming time compared with the control group, group administered with only coenzyme Q10 (Comparative Example 1), or group administered with only phosphatidylinositol (Comparative Example 2).

**[Table 2]**

| Group | Swimming time (average) (sec) | |
|---|---|---|
| | Week 0 | Week 3 |
| Control group | 214 | 307 |
| CoQ 10-administered group (Comparative Example 1) | 214 | 358 |
| Phosphatidylinositol-administered group (Comparative Example 2) | 212 | 333 |
| Group administered with CoQ10 + phosphatidylinositol (Test Example) | 214 | 394 |

### [Test Example 2]

Seven-week-old male Slc:SD rats were preliminarily fed for 7 days, and forced oral administration (repeated oral administration) was performed for 4 rats with phosphatidylinositol at 100 mg/kg/day for 1 week. Then, forced oral administration was performed with phosphatidylinositol at 100 mg/kg/day and oxidized coenzyme Q10 at 100 mg/kg/day at the same time (PI-administered group). 0, 2, 4, and 6 hours after the forced oral administration with both phosphatidylinositol and coenzyme Q10, the whole blood was collected from the postcaval vein using heparinized injection needles and syringes under diethyl ether anesthesia. Collected blood was immediately centrifuged (4°C, 3,000 rpm, 10 minutes) to separate plasma. The separated plasma samples were used for an analysis of coenzyme Q 10 by HPLC. As comparative examples, the above-mentioned procedures were performed for the group which was subjected to forced oral administration (repeated oral administration) with soybean lecithin at 500 mg/kg/day (100 mg/kg/day in terms of phosphatidylinositol) instead of phosphatidylinositol for 1 week and then to forced oral administration with both phosphatidylinositol at 100 mg/kg/day and oxidized coenzyme Q10 at 100 mg/kg/day at the same time (soybean lecithin-administered group), and the group which was subjected to forced oral administration with both phosphatidylinositol at 100 mg/kg/day and oxidized coenzyme Q10 at 100 mg/kg/day simultaneously without preliminary administration of phosphatidylinositol (PI non-administration group).
As shown in Tables 3 and 4, the coenzyme Q 10 concentrations in blood of the PI-administered group were found to be higher than those of the PI non-administration group, and it was found that phosphatidylinositol promoted the absorbability of coenzyme Q10 into the body. Meanwhile, the coenzyme Q9 oxidation ratios of the PI-administered group had a tendency to be lower than those of the PI non-administration group. The coenzyme Q10 concentration in blood of the soybean lecithin-administered group was slightly increased, but the coenzyme Q9 oxidation ratio (the value of the oxidized coenzyme Q9 based on total coenzyme Q9 (%)) was found to be about the same as that of the PI non-administration group.

**[Table 3]**

| | Average value of total CoQ10 concentration in blood (nM) | | |
|---|---|---|---|
| Time (hr) | PI-administered group | PI non-administration group | Soybean lecithin-administered group |
| 0 | 70 | 64 | 30 |
| 2 | 1,983 | 1,622 | 1,767 |
| 4 | 1,927 | 704 | 1,677 |
| 6 | 686 | 823 | 745 |

**[Table 4]**

| | Average value of CoQ9 oxidation ratio (nM) | | |
|---|---|---|---|
| Time (hr) | PI-administered group | PI non-administration group | Soybean lecithin-administered group |
| 0 | 24 | 23 | 25 |
| 2 | 14 | 21 | 17 |
| 4 | 13 | 18 | 14.4 |
| 6 | 7.4 | 15 | 11 |

### [Test Example 3]

Seven-week-old male ICR mice were preliminarily fed for 7 days, and both phosphatidylinositol (200 mg/kg/day) and oxidized coenzyme Q10 (100 mg/kg/day) were orally administered at the same time to ten rats repeatedly for 6 days. On day 6 of administration, the plunge time until fall from an accelerated rotarod was measured under conditions where the velocity reached 40 rpm in 6 minutes from 4 rpm of the initial velocity. On day 7 of administration, forced oral administration was performed with 15% ethanol at a dose of 2.0 g/kg, and 20 minutes after the administration of ethanol, the plunge time until fall from an accelerated rotarod was measured in the same way as day 6 under conditions where the velocity reached 40 rpm in 6 minutes from 4 rpm of the initial velocity. On the day before administration of ethanol, there was admitted no difference in the plunge time from the rotarod between the group administered with phosphatidylinositol at 200 mg/kg/day and the oxidized coenzyme Q10 at 100 mg/kg/day and the non-administration group. As shown in Table 5, the plunge time from the rotarod of the group administered with phosphatidylinositol at 200 mg/kg/day and oxidized coenzyme Q10 at 100 mg/kg/day after administration of ethanol was admitted to significantly increase compared with that of the non-administration group.

**[Table 5]**

| | Average plunge time (sec) | |
|---|---|---|
| | PI and coenzyme Q 10 -administered group | Non-administration group |
| Day 6 | 233 | 215 |
| Day 7 | 183 | 144 |

### Industrial Applicability

The anti-fatigue agent or physical endurance enhancer, or the functional food having an anti-fatigue effect or physical endurance-enhancing effect of the present invention can be suitably used in the field of drugs and/or foods.

## Claims

1. A composition comprising at least phosphatidylinositol and coenzyme Q10.

2. A composition according to Claim 1, wherein the content of the phosphatidylinositol in total phospholipids is 50 mol% or more.

3. A composition according to Claim 1 or 2, wherein the phosphatidylinositol is prepared by a method comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

4. A dietary supplement comprising the composition according to any one of Claims 1 to 3.

5. A functional food comprising the composition according to any one of Claims 1 to 3, and having an anti-fatigue effect and/or a physical endurance-enhancing effect.

6. A cosmetic comprising the composition according to any one of Claims 1 to 3.

7. An anti-fatigue agent and/or a physical endurance enhancer comprising the composition according to any one of Claims 1 to 3.

8. An agent for promoting absorption of coenzyme Q10 into a body, comprising at least phosphatidylinositol as an active ingredient.

9. An agent for promoting absorption of coenzyme Q10 into a body according to Claim 8, wherein the content of the phosphatidylinositol in total phospholipids is 50 mol% or more.

10. An agent for promoting absorption of coenzyme Q10 into a body according to Claim 8 or 9, wherein the phosphatidylinositol is prepared by a method comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

11. A functional food having an effect of promoting absorption of coenzyme Q10 into a body, comprising at least phosphatidylinositol as an active ingredient.

12. A functional food having an effect of promoting absorption of coenzyme Q10 into a body according to Claim 11, wherein the content of the phosphatidylinositol in total phospholipids is 50 mol% or more.

13. A functional food having an effect of promoting absorption of coenzyme Q10 into a body according to Claim 11 or 12, wherein the phosphatidylinositol is prepared by a method comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

14. A kit comprising an anti-fatigue agent and/or a physical endurance enhancer comprising phosphatidylinositol as an active ingredient and an anti-fatigue agent and/or a physical endurance enhancer comprising coenzyme Q10 as an active ingredient in combination for the purpose of simultaneous ingestion of the phosphatidylinositol and the coenzyme Q10.

15. A kit comprising an anti-fatigue agent and/or a physical endurance enhancer comprising phosphatidylinositol as an active ingredient and an anti-fatigue agent and/or a physical endurance enhancer comprising coenzyme Q10 as an active ingredient in one package for the purpose of simultaneous ingestion of the phosphatidylinositol and the coenzyme Q 10.

16. A kit according to Claim 14 or 15, wherein the content of the phosphatidylinositol in total phospholipids is 50 mol% or more.

17. A method of preparing phosphatidylinositol, comprising at least the following step of:
reacting a mixture of phospholipid derived from soybean with an enzyme which is derived from the genus *Candida* and is substantially incapable of decomposing the phosphatidylinositol and is capable of decomposing all of phosphatidylcholine, phosphatidylethanolamine, and phosphatidic acid to increase the content of the phosphatidylinositol in total phospholipids to 50 mol% or more.

18. A use of phosphatidylinositol and coenzyme Q10 for preparing a food, a cosmetic, or an anti-fatigue agent and/or a physical endurance enhancer each having an anti-fatigue effect and/or a physical endurance-enhancing effect.

19. A method of ameliorating fatigue and/or enhancing physical endurance, comprising simultaneously ingesting phosphatidylinositol and coenzyme Q10.

20. An alcohol metabolism-promoting agent comprising the composition according to any one of Claims 1 to 3.


SEQUENCE LISTING

<110> ASAHI KASEI PHARMA CORPORATION

<120>Dietary Supplement

<130> F107120WO

<140>
<141>

<150>JP 2006-315874
<151>2006-11-22

<150>JP 2007-007013
<151>2007-1-16

<160> 2

<170> PatentIn Ver. 2.1

<210> 1
<211> 549
<212> PRT
<213> Candida

<220>
<223>Inventor : Shinji. Koga

<400> 1
Met Glu Leu Ala Leu Ala Leu Leu Leu Ile Ala Ser Val Ala Ala Ala
1               5                   10                  15

Pro Thr Ala Thr Leu Ala Asn Gly Asp Thr Ile Thr Gly Leu Asn Ala
            20                  25                  30

Ile Ile Asn Glu Ala Phe Leu Gly Ile Pro Phe Ala Glu Pro Pro Val
        35                  40                  45

Gly Asn Leu Arg Phe Lys Asp Pro Val Pro Tyr Ser Gly Ser Leu Asp
    50                  55                  60

Gly Gln Lys Phe Thr Leu Tyr Gly Pro Leu Cys Met Gln Gln Asn Pro
65                  70                  75                  80

Glu Gly Thr Tyr Glu Glu Asn Leu Pro Lys Ala Ala Leu Asp Leu Val
                85                  90                  95

Met Gln Ser Lys Val Phe Glu Ala Val Leu Pro Leu Ser Glu Asp Cys
            100                 105                 110

Leu Thr Ile Asn Val Val Arg Pro Pro Gly Thr Lys Ala Gly Ala Asn
        115                 120                 125

Leu Pro Val Met Leu Trp Ile Phe Gly Gly Gly Phe Glu Val Gly Gly
    130                 135                 140

Thr Ser Thr Phe Pro Pro Ala Gln Met Ile Thr Lys Ser Ile Ala Met
145                 150                 155                 160

Gly Lys Pro Ile Ile His Val Ser Val Asn Tyr Arg Val Ser Ser Trp
                165                 170                 175

Gly Phe Leu Ala Gly Asp Glu Ile Lys Ala Glu Gly Ser Ala Asn Ala
            180                 185                 190

Gly Leu Lys Asp Gln Arg Leu Gly Met Gln Trp Val Ala Asp Asn Ile
        195                 200                 205

Ala Ala Phe Gly Gly Asp Pro Thr Lys Val Thr Ile Phe Gly Glu Leu
    210                 215                 220

Ala Gly Ser Met Ser Val Met Cys His Ile Leu Trp Asn Asp Gly Asp
225                 230                 235                 240

Asn Thr Tyr Lys Gly Lys Pro Leu Phe Arg Ala Gly Ile Met Gln Leu
                245                 250                 255

Gly Ala Met Val Pro Leu Asp Ala Val Asp Gly Ile Tyr Gly Asn Glu
            260                 265                 270

Ile Phe Asp Leu Leu Ala Ser Asn Ala Gly Cys Gly Ser Ala Ser Asp
        275                 280                 285

Lys Leu Ala Cys Leu Arg Gly Val Leu Ser Asp Thr Leu Glu Asp Ala
    290                 295                 300

Thr Asn Asn Thr Pro Gly Phe Leu Ala Tyr Ser Ser Leu Arg Leu Leu
305                 310                 315                 320

Tyr Leu Pro Arg Pro Asp Gly Val Asn Ile Thr Asp Asp Met Tyr Ala
                325                 330                 335

Leu Val Arg Glu Gly Lys Tyr Ala Asn Ile Pro Val Ile Ile Gly Asp
            340                 345                 350

Gln Asn Asp Glu Gly Thr Phe Phe Gly Thr Leu Leu Leu Asn Val Thr
        355                 360                 365

Thr Asp Ala Gln Ala Arg Glu Tyr Phe Lys Gln Leu Phe Val His Ala
    370                 375                 380

Ser Asp Ala Glu Ile Asp Thr Leu Met Thr Ala Tyr Pro Gly Asp Ile
385                 390                 395                 400

Thr Gln Gly Leu Pro Phe Asp Thr Gly Ile Leu Asn Ala Leu Thr Pro
                405                 410                 415

Gln Phe Lys Arg Ile Leu Ala Val Leu Gly Asp Leu Gly Phe Thr Leu
            420                 425                 430

Ala Arg Arg Tyr Phe Leu Asn His Tyr Thr Gly Gly Thr Lys Tyr Ser
        435                 440                 445

Phe Leu Leu Lys Gln Leu Leu Gly Leu Pro Val Leu Gly Thr Phe His
    450                 455                 460

Ser Asn Asp Ile Val Phe Gln Asp Tyr Leu Leu Gly Ser Gly Ser Leu
465                 470                 475                 480

Ile Tyr Asn Asn Ala Phe Ile Ala Phe Ala Thr Asp Leu Asp Pro Asn
                485                 490                 495

Thr Ala Gly Leu Leu Val Lys Trp Pro Glu Tyr Thr Ser Ser Leu Gln
            500                 505                 510

Leu Gly Asn Asn Leu Met Met Ile Asn Ala Leu Gly Leu Tyr Thr Gly
        515                 520                 525

Lys Asp Asn Phe Arg Thr Ala Gly Tyr Asp Ala Leu Phe Ser Asn Pro
    530                 535                 540

Pro Leu Phe Phe Val
545

<210> 2
<211> 549
<212> PRT
<213> Candida

<220>
<223>Inventor : Shinji, Koga

<400> 2
Met Lys Leu Ala Leu Ala Leu Leu Leu Ile Ala Ser Val Ala Ala Ala
1               5                   10                  15

Pro Thr Ala Lys Leu Ala Asn Gly Asp Thr Ile Thr Gly Leu Asn Ala
            20                  25                  30

Ile Ile Asn Glu Ala Phe Leu Gly Ile Pro Phe Ala Glu Pro Pro Val
        35                  40                  45

Gly Asn Leu Arg Phe Lys Asp Pro Val Pro Tyr Ser Gly Ser Leu Asn
    50                  55                  60

Gly Gln Lys Phe Thr Leu Tyr Gly Pro Leu Cys Met Gln Gln Asn Pro
65                  70                  75                  80

Glu Gly Thr Phe Glu Glu Asn Leu Gly Lys Thr Ala Leu Asp Leu Val
                85                  90                  95

Met Gln Ser Lys Val Phe Gln Ala Val Leu Pro Gln Ser Glu Asp Cys
            100                 105                 110

Leu Thr Ile Asn Val Val Arg Pro Pro Gly Thr Lys Ala Gly Ala Asn
        115                 120                 125

Leu Pro Val Met Leu Trp Ile Phe Gly Gly Gly Phe Glu Ile Gly Ser
    130                 135                 140

Pro Thr Ile Phe Pro Pro Ala Gln Met Val Thr Lys Ser Val Leu Met
145                 150                 155                 160

Gly Lys Pro Ile Ile His Val Ala Val Asn Tyr Arg Val Ala Ser Trp
                165                 170                 175

Gly Phe Leu Ala Gly Asp Asp Ile Lys Ala Glu Gly Ser Gly Asn Ala
            180                 185                 190

Gly Leu Lys Asp Gln Arg Leu Gly Met Gln Trp Val Ala Asp Asn Ile
        195                 200                 205

Ala Gly Phe Gly Gly Asp Pro Ser Lys Val Thr Ile Phe Gly Glu Leu
    210                 215                 220

Ala Gly Ser Met Ser Val Leu Cys His Leu Ile Trp Asn Asp Gly Asp
225                 230                 235                 240

Asn Thr Tyr Lys Gly Lys Pro Leu Phe Arg Ala Gly Ile Met Gln Leu
                245                 250                 255

Gly Ala Met Val Pro Leu Asp Pro Val Asp Gly Thr Tyr Gly Asn Glu
            260                 265                 270

Ile Tyr Asp Leu Phe Val Ser Ser Ala Gly Cys Gly Ser Ala Ser Asp
        275                 280                 285

Lys Leu Ala Cys Leu Arg Ser Ala Leu Ser Asp Thr Leu Leu Asp Ala
    290                 295                 300

Thr Asn Asn Thr Pro Gly Phe Leu Ala Tyr Ser Ser Leu Arg Leu Leu
305                 310                 315                 320

Tyr Leu Pro Arg Pro Asp Gly Lys Asn Ile Thr Asp Asp Met Tyr Lys
                325                 330                 335

Leu Val Arg Asp Gly Lys Tyr Ala Ser Val Pro Val Ile Ile Gly Asp
            340                 345                 350

Gln Asn Asp Glu Gly Thr Ile Phe Gly Leu Leu Leu Leu Asn Val Thr
        355                 360                 365

Thr Asn Ala Gln Ala Arg Ala Tyr Phe Lys Gln Leu Phe Ile His Ala
    370                 375                 380

Ser Asp Ala Glu Ile Asp Thr Leu Met Ala Ala Tyr Pro Gln Asp Ile
385                 390                 395                 400

Thr Gln Gly Leu Pro Phe Asp Thr Gly Ile Phe Asn Ala Ile Thr Pro
                405                 410                 415

Gln Phe Lys Arg Ile Leu Ala Val Leu Gly Asp Leu Ala Phe Ile His
            420                 425                 430
Ala Arg Arg Tyr Phe Leu Asn His Phe Gln Gly Gly Thr Lys Tyr Ser
        435                 440                 445
Phe Leu Leu Lys Gln Leu Leu Gly Leu Pro Ile Met Gly Thr Phe His
    450                 455                 460
Ala Asn Asp Ile Val Trp Gln Asp Tyr Leu Leu Gly Ser Gly Ser Val
465                 470                 475                 480
Ile Tyr Asn Asn Ala Phe Ile Ala Phe Ala Thr Asp Leu Asp Pro Asn
                485                 490                 495
Thr Ala Gly Leu Leu Val Asn Trp Pro Lys Tyr Thr Ser Ser Leu Gln
            500                 505                 510
Leu Gly Asn Asn Leu Met Met Ile Asn Ala Leu Gly Leu Tyr Thr Gly
        515                 520                 525
Lys Asp Asn Phe Arg Thr Ala Gly Tyr Asp Ala Leu Met Thr Asn Pro
    530                 535                 540
Leu Leu Phe Phe Val
545